# EUROPEAN PATENT SPECIFICATION

(11) **EP 0 471 837 B1**
(45) Date of publication and mention of the grant of the patent: **15.01.1997**
(21) Application number: 91908132.3
(22) Date of filing: 01.03.1991
(51) Int. Cl.: A61M 39/02, A61M 39/06

(54) **IMPLANTABLE INFUSION DEVICE**
IMPLANTIERBARE INFUSIONSVORRICHTUNG
DISPOSITIF DE PERFUSION IMPLANTABLE

(30) Priority: 01.03.1990 US 487541; 18.06.1990 US 539793; 15.02.1991 US 654661
(43) Date of publication of application: 26.02.1992
(73) Proprietor: Ensminger, William Darrel, Ann Arbor, Michigan 48103 (US); Andrew, James Carroll, Ann Arbor, Michigan 48103 (US); Knol, James Alan, Ann Arbor, Michigan 48103 (US)
(72) Inventor: Ensminger, William Darrel, Ann Arbor, Michigan 48103 (US); Andrew, James Carroll, Ann Arbor, Michigan 48103 (US); Knol, James Alan, Ann Arbor, Michigan 48103 (US)
(74) Representative: Senior, Alan Murray
(86) International application number: PCT/US91/01414
(87) International publication number: WO 91/12838

(56) References cited:
- EP-A- 0 309 092
- US-A- 4 430 081
- US-A- 4 447 237
- US-A- 4 569 675
- US-A- 4 673 394
- US-A- 4 710 167
- US-A- 4 781 693
- US-A- 4 857 062

## Description

### FIELD OF THE INVENTION

This invention relates to a device for introducing a flexible or non-flexible filament into a patient such as a catheter for infusing a therapeutic agent to a desired site within the patient or withdrawing a fluid, and more particularly, to such a device which is implanted such that no portion is transcutaneous. Its access portion is subcutaneous but designed so as to facilitate repeated access by the percutaneous route.

### BACKGROUND AND SUMMARY OF THE INVENTION

In current human and animal medical practice, there are numerous instances where therapeutic agents must be delivered to a specific organ or a tissue within the body. An example is the infusion of chemotherapy into a central vein on a recurring basis over a lengthy treatment period for widespread sites of malignant tumor. Without an infusion device for intravenous drug infusion, multiple vein punctures over a lengthy period results in progressive thrombosis, venous sclerosis, and destruction of small diameter peripheral vessels. In other cases, it may be desirable to infuse chemotherapy to a localized malignant tumor site. It may be difficult or impossible to deliver an agent specifically to such a site on a regular repetitive basis without surgically implanting an infusion system. Similarly, repeated arterial access is occasionally needed for injection of an X-ray dye or contrast agent into an artery for diagnostic purposes. In other situations, there is a need to remove a body fluid repetitively for analysis from a remote body site. Finally, sensing and physiological measuring devices incorporated into small diameter catheters and small diameter optical fibers are increasingly being utilized for monitoring body processes and could be more easily implemented through a properly designed access device with an adequate internal diameter.

In prior medical practice, percutaneous catheters have been used to provide vascular or organ access for drug therapy or removing body fluids. Although such systems generally performed in a satisfactory manner, numerous problems were presented by such therapy approaches, including the substantial care requirements by patients, e.g. dressing changes with sterile techniques, a significant rate of infection of the catheter because of its transcutaneous position, and a high rate of venous thrombosis, particularly if the catheter was located within an extremity vein.

Implantable infusion devices or "ports" have recently become available and are a significant advance over transcutaneous catheters. Presently available infusion ports have a number of common fundamental design features. The ports themselves comprise a housing which forms a reservoir which can be constructed from a variety of plastic or metal materials. A surface of the reservoir is enclosed by a high-density, self-sealing septum, typically made of silicone rubber. Connected to the port housing is an outflow catheter which communicates which a vein or other site within the patient where it is desired to infuse therapeutic agents. Implantation of such devices generally proceeds by making a small subcutaneous pocket in the patient under local anaesthesia. The internal outflow catheter is tunnelled to the desired infusion site. When the patient desires to infuse or remove material through the port, a hypodermic needle is used which pierces the skin over the infusion port and is placed into the port. Such an implantable infusion device is shown in EP-A-0 309 092.

Although presently available implantable infusion ports generally operate in a satisfactory manner,they have a number of shortcomings. Since these devices rely on a compressed rubber septum for sealing, there are limitations in the diameter of needles which can be used to penetrate he septum, since large diameter needles can seriously damage the septum. These diameter limitations severely restrict the flow rate of fluids passing through the port. In cases where it is desirable to infuse drugs using a flexible external inflow catheter, the catheter must be fed through the needle which penetrates the septum. Such catheters have an extremely small inside diameter and, therefore, impose severe limitations on fluid flow rate.

For prolonged infusion using a conventional port, the infusion needle is taped to the patient's skin to hold it in position. Conventional ports do not allow the needle to penetrate deeply into the port so that a small displacement of the needle can cause it to be pulled from the port. In cases where locally toxic materials are being infused, extravasation of such materials can cause local tissue damage which can lead to a requirement for corrective surgery such as skin grafting or removal of tissue.

Presently available implantable drug infusion devices must also have a significant size to provide an acceptable target surface area for the physician who must locate the port and penetrate the septum properly with a needle. The port housing becomes bulky as the septum size increases since structure is required to maintain the septum in compression to provide self-sealing after the needle is removed. Moreover, presently available infusion ports are difficult to clear if thrombosis occurs within them or in the implanted outflow catheter, since it is difficult, if not impossible, to feed a cleaning wire through the penetrating hypodermic needle in a manner which will clear the infusion device and the internal outflow catheter. Present infusion ports have a retained volume beneath the self-sealing septum which increases the volume of drug which must be administered to enable a desired quantity to reach the infusion site. This retained volume also poses problems when a physician desires to deliver drugs to the same infusion site which are incompatible when mixed. In addition, when it is desired to withdraw blood through the port, the retained volume of the prior art infusion ports comprises an area where blood clotting can occur, thus interfering with future access to the site. And, finally, for the present infusion ports, there is a risk that the physician attempting to pierce the port septum will not properly enter it, leading to the possibility of extravasation which can cause significant undesirable consequences.

US-A-4,447,237 discloses a totally implantable patient infusion device for permitting access to an internal catheter by a filament comprising a housing having a funnel shaped entrance orifice having a decreasing cross-sectional area which leads to a focus area, the housing further having a passageway communicating said focus area with an exit orifice, said housing causing the filament introduced into the entrance orifice to be directed to said focus area and to enter said passageway and a valve positioned within the housing passageway which normally remains closed to provide resistance to flow of fluid through said valve, yet opens to permit the filament to pass though the valve enabling the filament to communicate with the internal catheter through said exit orifice, means being provided for mounting the infusion device subcutaneously. However, such an implantable access device is only capable of being used with a special accessing instrument such as a rigid cannula and trocar and requires an entrance orifice which is carefully matched to the diameter of a special rigid trocar. The sealing action between the inserted rigid filament and the valve is dependent entirely upon the application of radial compressive force upon the rigid instrument having a specific predetermined diameter.

It is now proposed, according to the present invention, to provide an implantable patient infusion device adapted to be totally implanted within the body of a patient to permit access to an internal catheter either by a flexible or by a non-flexible filament such as a needle, external catheter, wire or optical fibre, the device including a housing having a funnel-shaped entrance orifice with a decreasing cross-sectional area leading to a focus area, and an articulating valve in a passageway communicating said focus area with an exit orifice, said funnel-shaped entrance orifice having an open area four or more times greater than the cross-sectional area of the passageway, and being arranged to cause the filament, when introduced into said entrance orifice, to be directed to said focus area to enter said passageway and to engage said valve at a predetermined location thereon, said valve being adapted normally to remain closed to provide resistance to flow of fluids through said passageway, yet openable upon engagement with said filament to permit said filament to pass through said articulating valve and to enable said filament to communicate with an internal catheter through said exit orifice, said articulating valve comprising at least one deflectable valve element arranged to be articulated and thereby deflected both longitudinally and laterally of the direction of movement of the filament through said articulating valve upon opening of said valve by the filament.

The present invention thus relates to a family of implantable infusion ports which provide numerous enhancements over the prior art devices. In accordance with the invention, the infusion ports provided incorporate a funnel shaped entrance orifice which narrows down to a reduced diameter guide passageway. The guide passageway terminates at an internal cavity which retains a catheter valve. Instead of using a radially compressed slit valve, where compression is provided by an element apart from the slit valve itself, as in US-A-4447237, the present invention utilizes an articulating valve, preferably a multi-element leaflet valve assembly. Such an articulating valve is readily reusable without being damaged or having its efficiency harmed by the passage of a sharp needle and can also pass a more flexible member which is not so restricted to a specific diameter than when using a compressed rubber septum as the valve. The port of the invention also has an exit passageway which is connected to an implanted outflow catheter.

Several embodiments of this invention are intended to be used by inserting a blunt instrument through the skin and into the port entrance orifice which introduces a filament such as a catheter into the port. The infusion port in accordance with other embodiments of the present invention are adapted to be used in conjunction with a sharp hypodermic access needle of conventional design which may be used by itself for infusion or fluid withdrawal, or with an external inflow catheter having a needle fed through it (or vice versa) allowing a catheter to be put in position within the infusion port or fed into the implanted catheter for infusion or withdrawal of fluid. The entrance orifice has a metal surface which guides the needle to the guide passageway. The reduced diameter guide passageway of the port housing can be used to accurately align the access needle and/or catheter to strike the catheter valve at a desired area so that a needle can be used to penetrate the catheter valve repeatedly without impairing the function of the valve.

According to another group of preferred features of this invention, additional features of infusion ports are described. One area of potential improvement for some purposes is the provision of a port designed for implantation in a patient's arm which has an access passageway for an inserted needle. The body of this port is angled upwardly slightly to facilitate access. Such an angled infusion port can also feature modifications to the entrance orifice to again further enhance the ability to access the implanted port. This application further describes a valving concept for an implanted port which provides a high degree of resistance to body fluid leakage through the port and further provides a relatively low level of friction upon insertion of an external catheter, with a relatively higher degree of friction upon withdrawal of the catheter. This difference in resistance aids both in insertion of the catheter and in maintaining the catheter in an inserted condition within the implanted port.

This specification also describes port design features which are best embodied in a port in which the entrance funnel is in a plane generally parallel to the mounting base of the port (i.e. the accessing needle penetrates perpendicular to the mounting base). One improvement for such ports is the provision of a physical feature such as a projecting lug, flange or other protuberance which enables the clinician to determine the orientation of the implanted port through tactile examination. By knowing the port orientation, the needle and introduced filament can often be more readily inserted into the port. This series of ports also known as "chest wall" ports (named for a preferred usage) also feature a funnel-shaped entrance orifice having a progressively changing included angle. The orifice starts at its outer periphery with a relatively shallow included angle which increases toward its center. This progressive change in cone angle provides two significant benefits. First, it results in a port which has a relatively shallow tunnel which reduces the distance between the skin surface and the catheter valve which seals around the introduced catheter or the filament and also serves to better orient and hold the introducing needle. Several of the ports according to this specification also feature means for stopping the introduced needle before reaching the catheter valve but permit the introduced catheter to pass through the catheter valve.

The infusion ports of this invention are implanted in the same general manner as prior art devices. When the physician desires to infuse a therapeutic agent, remove a body fluid, or have vascular access, a filament such as a catheter is introduced into the port. The entrance orifice guides the introduced catheter or needle into a proper "docking" position with the articulating catheter valve. By pushing on the externally introduced filament, it is forced through the catheter valve, thereby providing an open communication pathway for the infusion of therapeutic agents, extraction of body fluids, introduction of an optical fiber, clearing by a wire, etc. The introduced filament can be fed into the outflow catheter to any extent desired. In the case of introducing a flexible catheter, a guide wire can be inserted into the external catheter to increase its rigidity. The convenient access to the port and internal outflow catheter enables these elements to be cleared with a clearing wire so that they can always be cleared, avoiding the problem of permanent impaction of prior art devices. In addition, the ability to feed a guide wire into the infusion port and internal catheter of this invention enables the internal catheter to be repositioned using a bent or "steerable" guide wire.

The infusion ports having an articulating catheter valve of this invention possess the advantage that they have a very small reservoir or "dead space", meaning that virtually all of the infused fluid is throughput to the desired infusion site. This invention, therefore, facilitates infusion of incompatible materials in a serial fashion since very little of the previously infused fluid remains in the device when a subsequent infusion is carried out. This invention also facilitates simultaneous infusion of incompatible materials by using a multi-lumen catheter.

Another embodiment of the present invention has an infusion port which is configured such that a line normal to the plane formed by the entrance orifice is nearly at a right angle to the exit passageway. The port access opening guides an introduced filament toward and into the outflow catheter. Other aspects of the present invention relate to providing a reservoir within an infusion port for containing an antimicrobial fluid, offering enhanced protection against introduced infection. This invention is further related to various means of securely fastening an outflow catheter to an infusion port.

Additional benefits and advantages of the present invention will become apparent to those skilled in the art to which this invention relates from the subsequent description of the preferred embodiments and the appended claims, taken in conjunction with the accompanying drawings.

### BRIEF DESCRIPTION OF THE DRAWINGS

Figure 1 is a cross-sectional view of an infusion port in accordance with a first embodiment of the present invention shown with an external catheter and obturator inserted though a leaflet type catheter valve which is a first embodiment of an articulating valve.

Figure 2 is a pictorial view of a skin punch which may be used to make an incision into a patient's skin to permit insertion of an external catheter.

Figure 3 is an illustration of a stab wound formed by the skin punch shown in Figure 2.

Figure 4 is a pictorial view of the leaflet valve used with the infusion port of Figure 1.

Figure 5 is a frontal view of a cup type catheter valve which is an alternate embodiment of an articulating valve.

Figure 6 is a cross-sectional view of the valve of Figure 5 shown in a closed position.

Figure 7 is a cross-sectional view from Figure 5 showing the catheter valve in a partly open position.

Figure 8 is a sectional view taken from Figure 5 showing the catheter valve in a fully open position permitting passage of an introduced catheter.

Figure 9 is a frontal view of a valve of the ball-and-seat variety which is an alternate embodiment of an articulating valve.

Figure 10 is a cross-sectional view from Figure 9 showing the ball valve in a fully closed condition.

Figure 11 is a cross-sectional view taken from Figure 9 showing the ball valve in a fully opened condition.

Figure 12 is a cross-sectional of an embodiment of this invention similar to Figure 1, illustrating that an external introduced catheter may be placed well into the internal outflow catheter of the infusion port.

Figure 13 is a cross-sectional view similar to Figure 12 except showing the introduced catheter being fed through the infusion port such that its terminal end is beyond the terminal end of the internal outflow catheter.

Figure 14 is a pictorial view of an infusion port in accordance with a second embodiment of this invention shown providing a change in angle for the external introduced catheter.

Figure 15 is a cross-sectional view taken to the infusion port of Figure 14 showing the infusion port in use and showing an external introduced catheter in position for infusion of the patient.

Figure 16 is a partial cross-sectional view of an infusion port in accordance with a third embodiment of this invention shown employing a pair of separated leaflet valves which provide a reservoir for an antimicrobial fluid which provides enhanced resistance against infection.

Figure 17 is a cross-sectional view of an infusion port which, although not in accordance with this invention in that it employs a conventional rubber septum does show means of guiding a catheter or guide wire through a bend and into or beyond the port exit orifice and which may be used with infusion devices embodying the invention.

Figure 18 is an infusion port having an elliptically shaped entrance mouth.

Figure 19 is a side view of the infusion port shown in Figure 18.

Figure 20 is a cross-sectional view illustrating a manner of connecting an internal outflow catheter to an infusion port incorporating an annular chamber for receiving the outflow catheter.

Figure 21 is a cross-sectional view of another means of attaching an internal outflow catheter to an infusion port, in which the catheter is placed over a smooth cylindrical surface and a compression ring is slid onto the junction.

Figure 22 is a cross-sectional view of still another approach toward connecting an internal outflow catheter to an infusion port incorporating a barbed nipple on the infusion port and a compression ring.

Figure 23 is another means for attaching an internal outflow catheter to an infusion port incorporating an interlocking compression ring.

Figure 24 is a pictorial view of an infusion port shown attached to an internal catheter.

Figure 25 illustrates an access needle with an external catheter being used to penetrate the infusion port shown in Figure 24.

Figure 26 is an exploded pictorial view of the infusion port illustrated in Figure 25 shown with an optional elastic ring sealing disc for use with the leaflet valve elements.

Figure 27 is a cross-sectional view taken from Figure 24 showing the internal construction of the infusion port.

Figure 28 is a frontal view of the elastic leaflet valve elements as shown in Figures 26 and 27.

Figure 29 is a pictorial view of an angled infusion port.

Figure 30 is a side view of the port shown in Figure 29 shown implanted within a patient and being accessed by a penetrating needle.

Figure 31 is a pictorial view of an eighth embodiment of an infusion port.

Figure 32 is a cross-sectional view taken along line 32-32 from Figure 30.

Figure 33 is an exploded pictorial view of the catheter valve of the port shown in Figures 31 and 32.

Figure 34 is an enlarged cross-sectional view similar to Figure 32 but showing an accessing needle being introduced into the port.

Figure 35 is a partial cross-sectional view showing the accessing needle and catheter being more fully inserted into the port.

Figure 36 is a partial cross-sectional view showing the introduced catheter penetrating the valve assembly of the port.

Figure 37 is a partial pictorial view showing an introduced catheter completely passing through the articulating valve and in a a proper docking position with the port for material infusion.

Figure 38 is an exploded pictorial view of another embodiment of an articulating catheter valve according to this invention.

Figure 39 is an exploded pictorial view of another alternative embodiment of an articulating catheter valve.

### DETAILED DESCRIPTION OF THE PREFERRED EMBODIMENTS

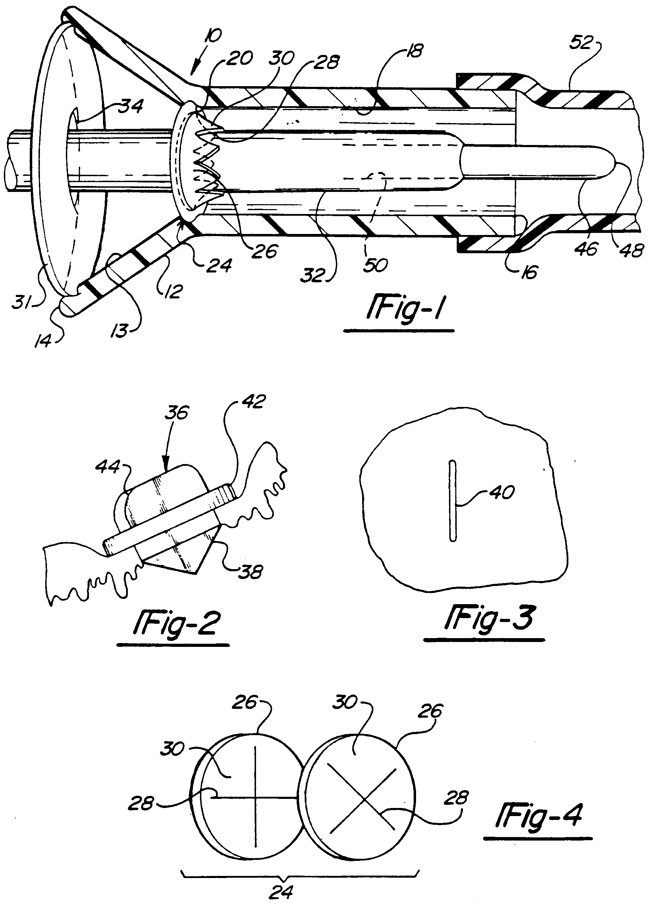

An infusion port in accordance with a first embodiment of this invention is shown in figure 1 and is generally designated there by reference number 10. Infusion port 10 generally comprises housing 12 defining an entrance orifice 14, an inside cavity 13 which funnels down to base 20, with an exit orifice 16, and an elongated passageway 18 extending between the external orifice base, and exit orifice 16. In the embodiment shown, infusion port housing 12 is rotationally symmetrical about a central longitudinal axis passing through passageway 18. As is evident from Figure 1, the diameter of entrance orifice 14 is preferably several times greater than the internal diameter of passageway 18 (i.e. an area difference four times or more). The entire housing 12 can be formed in one piece from numerous polymeric materials or metals which are compatible with human or animal implantation.

Positioned within passageway 18 is an articulating valve in the form of a leaflet valve assembly 24, which is also shown in an exploded fashion in Figure 4. Leaflet valve assembly 24 is comprised of one or more thin elastic disks 26 made, for example, from silicone rubber having one or more radial slits 28 cut through them. In the embodiment shown in Figures 1 and 4, two disk 26 are provided, each having two slits with a right angle between them so that each defines four leaves 30. The disks 26 are oriented and stacked against one another so that slits 28 of both the disks are angularly misaligned. This misalignment is intentionally provided to enhance the sealing characteristics of valve assembly 24 when it is in its normal closed position, as shown in Figure 4. Numerous other configurations for valve disk 26 can be provided, such as those incorporating any number of additional slits having various numbers of leaves.

The embodiment of infusion port 10 shown in Figure 1 includes an optional thin rubber septum 31 which acts to shield entrance orifice 14. When a foreign object is implanted in a human, the body often develops fibrous tissue around the object. If an exposed concave pocket is present, such as an open entrance orifice 14, the pocket could possibly become filled with such fibrous tissue. The development of this tissue, should such occur in a patient, might restrict access into the port, and potentially could interfere with the catheter valve function. Therefore, septum 31 provided which is pre-slit at 34 to allow the introduced external filament to easily penetrate the septum. Septum 31 does not, however, provide a fluid-tight barrier as in prior art infusion ports which have self-sealing characteristics and is easily penetrated by a blunt instrument. The provision of septum 31 prevents tissue growth inside the housing cavity and also enables the region of housing between entrance orifice 14 and leaflet valve assembly 24 to act as a reservoir for the retention of an antimicrobial fluid which aids in preventing the invasion of infectious agents during the use of infusion port 10.

In use, infusion port 10 is surgically positioned subcutaneously within the patient and mounted to suitable support tissue using conventional mounting techniques, such as sutures or surgical staples. Internal outflow catheter 52 is tunneled to the desired site in the body. When access is desired for the infusion of therapeutic agents, for the sampling of body fluids or for the introduction of physiological sensing elements (electrical or optical transducers, etc), a flexible external catheter 32 (or other filament) is introduced into infusion port 10, as shown in Figure 1. Insertion of external catheter 32 can be facilitate using skin punch 36 as shown in Figure 2. Skin punch 36 includes a pointed flat blade 38 having a width sufficient to make a desired length of an incision 40 shown in Figure 3. Skin punch 36 includes a radially extending flange 42 which limits the depth of the incision 40. Tab 44 provides a convenient means for holding and using skin punch 36. Once external catheter 32 is introduced through stab wound 40, if passes into entrance orifice 14 and is guided by the funnel shaped configuration of the housing cavity into orientation with leaflet valve assembly 24. Continued insertion of external catheter 32 causes the external catheter to penetrate leaflet valve assembly 24, causing deflection of valve leaves 30.

In cases where external catheters 32 are used which are quite flexible, it is necessary to provide localized stiffening of the introduced catheter to facilitate its introduction through the stab wound and into the proper docking position with leaflet valve assembly 24. For such cases, a semi-rigid guide wire or obturator 46 having a blunt end 48 can be used which is inserted through the internal passageway 50 of catheter 32.

Leaflet valve assembly 24 is relatively insensitive to the use of various diameters of external catheter 32, thus providing flexibility for the physician. Furthermore, the characteristics of leaflet valve assembly 24 are such that once external catheter 32 is inserted through the valve, the valve does not exert a large radially inward compressive force on the catheter, thus preventing collapsing of the catheter which would seal off internal passageway 50. However, it does provide sufficient friction on the external catheter to stabilize its position.

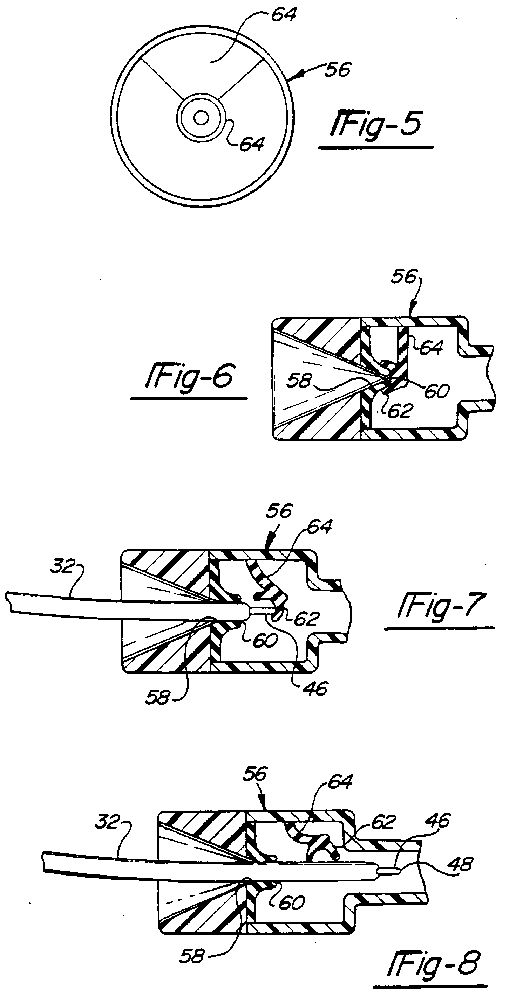

Figure 5 illustrates another embodiment for an articulating valve in the form of a cup-type catheter valve generally designated by reference number 56. Valve 56 is another articulating type valve which can be used as a replacement for leaflet valve assembly 24 shown in Figure 1. For this embodiment, a valve passageway 58 is formed which has a generally conically shaped exit nipple 60. A cup shaped closure valve 62 is provided which is supported in cantilever fashion by arm 64 which normally biases the cup closure valve into sealing engagement with exit nipple 60, as shown in Figure 6. Figure 7 illustrates catheter valve 56 when external catheter 32, reinforced with obturator 46 is initially penetrating valve passageway 58. During this process, external catheter 32 pushes cup closure valve 62 out of sealing engagement with valve nipple 60. Figure 8 illustrates the orientation of the elements of cup catheter valve 56 once external catheter 32 is fully introduced into the infusion port.

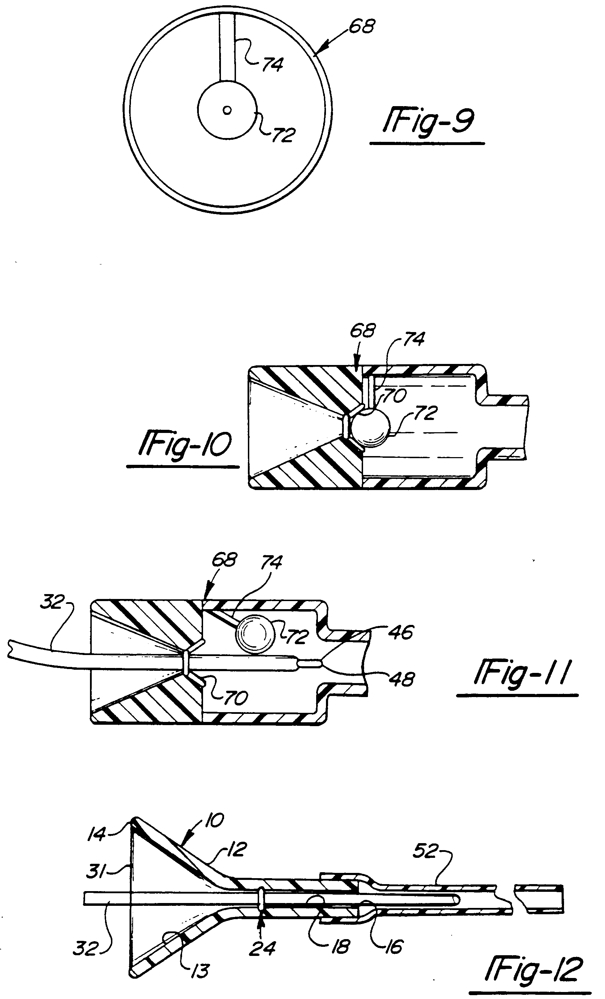

Figure 9 illustrates another embodiment for an articulating valve in the form of a ball-and-seal valve, generally designated by reference number 68. Ball-and-seat valve 68 defines a conical ball seat 70 with ball closure valve 72 which is normally biased into sealing engagement with the ball seat by arm 74. Operation of ball-and-seat valve 68 is similar to the operation of cup catheter valve 56 previously described. In both cases, external catheter 32 (or another filament), which may be stiffened by an obturator 46, physically unseats the valve element to permit passage of the external catheter.

Although the leaflet, cup, and ball-in-socket catheter valves described previously differ in their construction, each can be described as an "articulating" valve in that the introduced filament is accurately guided into an insertion area for the valve and deflects the valve in a predictable and repeatable manner to permit passage of a catheter or other filament. These valve types are distinguishable over prior art septums which are penetrated at random locations and do not define a predictable and defined passageway for a penetrating needle which cuts through and physically damages the septum. Applicants submit that there are numerous additional articulating valve designs which achieve these desired characteristics and are fully applicable to the infusion ports of the present invention.

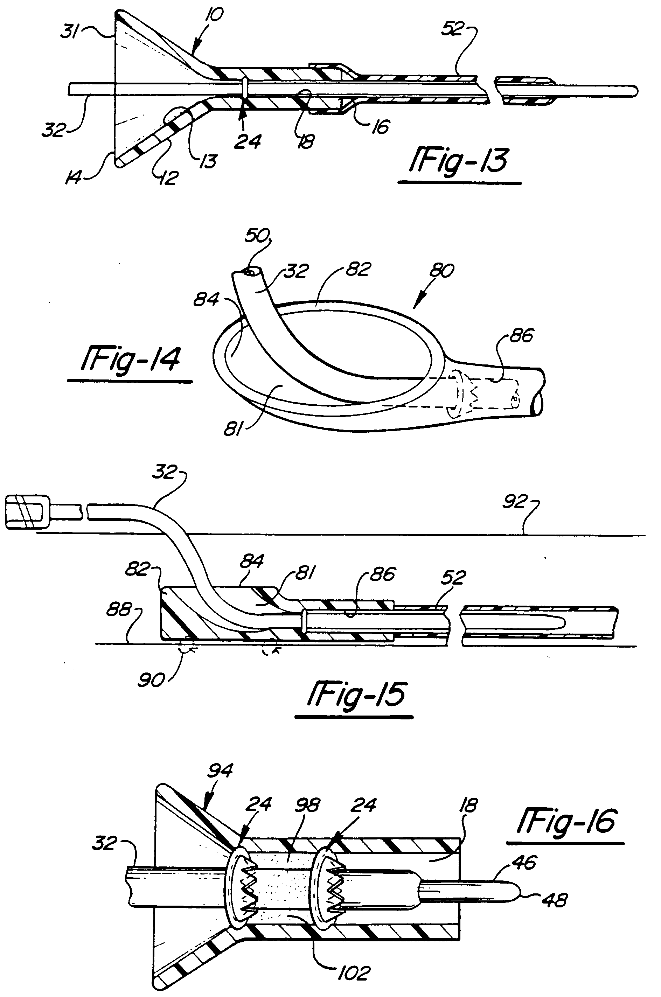

Figure 12 illustrates infusion port 10 described previously and shows that once external catheter 32 penetrates leaflet valve assembly 24 (or any other type of articulating valve used), the external catheter can be positioned at any desired point along internal outflow catheter 52. Figure 13 is a view similar to Figure 12 but shows that external catheter 32 can be fed through infusion port 10 so that its terminal end extends beyond that of internal catheter 52. This feature allows infusion port 10 to be readily adapted for angiography and angioplasty procedures.

Now with reference to Figures 14 and 15, a second embodiment of an infusion port according to this invention is shown which is generally designated by reference number 80. Infusion port 80 differs principally from infusion port 10 in that the internal cavity 81 of housing 82 is in the shape of a bent or twisted funnel or horn such that a line normal to the plane defined by entrance orifice 84 is generally at a right angle to the longitudinal central axis of exit passageway 86. Like the first embodiment, infusion port 80 employs an articulating catheter valve, such as a leaflet valve assembly 24 as previously described.

Infusion port cavity 81 has a smooth inside surface which is shaped to have a decreasing cross sectional area from entrance orifice 84 to exit passageway 86 for guiding external catheter 32 into registry with the exit passageway. The configuration of infusion port 80 is desirable where a large target area is needed which is generally parallel to the surface of the patients skin overlying the device. In all other respects, infusion port 60 is constructed and used in the manner consistent with that of infusion port 10 previously described. Figure 15 provides an illustration of infusion port 80 in use for infusing a patient. Port 80 is shown fastened to support tissue 88 by sutures 90 below skin 92 of the patient.

Figure 16 is a partial sectional view of an infusion port 94 according to a third embodiment of this invention. This embodiment differs from those described previously in that a pair of leaflet valve assemblies 24 is provided along internal passageway 18 to define an enclosed internal cavity 98. Internal cavity 98 is provided so that an antimicrobial solution 102 can be retained as a means of inhibiting the introduction of infectious agents into the patient through the process of infusion.

Figure 17 illustrates an infusion port which is designated by reference number 106. Like the second embodiment shown in Figure 14, infusion port housing 108 has an internal cavity 109 which causes an external catheter or other filament to undergo a right angle bend as it is fed into the device. However, infusion port 106 does not incorporate an articulating valve, but rather uses the conventional approach of using a compressed rubber septum 110. In use of this embodiment, a hypodermic needle 112 penetrates septum 110 and a small diameter catheter 114 is fed through needle 112. As discussed previously in connection with Figure 14, the internal surface configuration of housing cavity 109 causes catheter 114 to be guided into and through passageway 116, and if desired, into the attached internal catheter (not shown). This embodiment also provides the advantages that a guide wire can be fed through needle 112 to clear thrombosis or other obstructions occurring within the device or in the attached internal catheter.

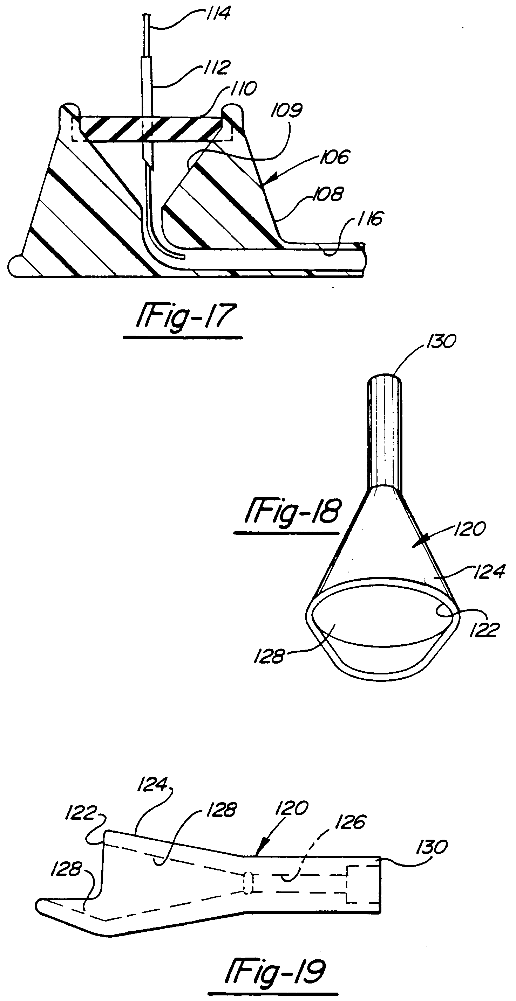

Figures 18 and 19 illustrate an infusion port 120 according to this invention whereby an entrance opening 122 can form a generally elliptical configuration such that the target area for the infusion port has is greatest area when entering the device from a direction between alignment with the exit passageway 126, or at right angle to exit passageway. In other words, a line normal to entrance opening 122 forms an obtuse angle to the axis of exit passageway 126. Like the prior embodiments, housing 124 has a smooth internal surface which is shaped to guide an introduced cavity 128 with external catheter into exit passageway 126.

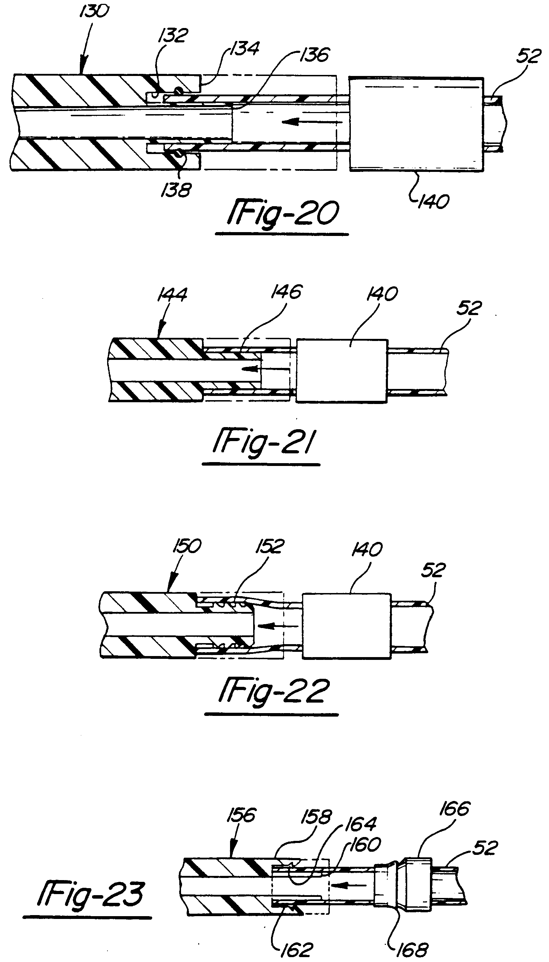

Figures 20 through 23 illustrate various means for attaching an internal outflow catheter 52 to an infusion port. For the embodiment of Figure 20, the infusion port features an exit end 130 defining an annular gap 132 formed between an outer tubular portion 134 of the exit outlet 134 and an inner tubular portion 136. Outflow catheter 52 is slid onto inner tube 136 and into annular gap 132. Sealing means such as a gasket or O-ring 138 can be provided to enhance the integrating of the fluid tight connection. Compression ring 140 can be used which is slid onto the connection as shown in Figure 20 to exert a compressive force on outflow catheter 52, further securing it to the infusion port. Compression ring 140 also acts as a stress reliever to prevent kinking of the outflow catheter 52 at its connection point to the infusion port.

Figure 21 illustrates another means for connecting outflow catheter 52 to an infusion port. In this embodiment, exit end 144 has a reduced diameter projecting nipple 146 which outflow catheter 152 is slid over. Like the embodiment shown in Figure 20, compression ring 140 is provided which is slid onto the connection with Figure 20.

Figure 22 illustrates an infusion port exit end 150 which features reversibly oriented barbs 152 which serve to securely engage the inner surface of outflow catheter 52. Again, compression ring 140 is used to enhance the security of the connection of the outflow catheter to exit end 150.

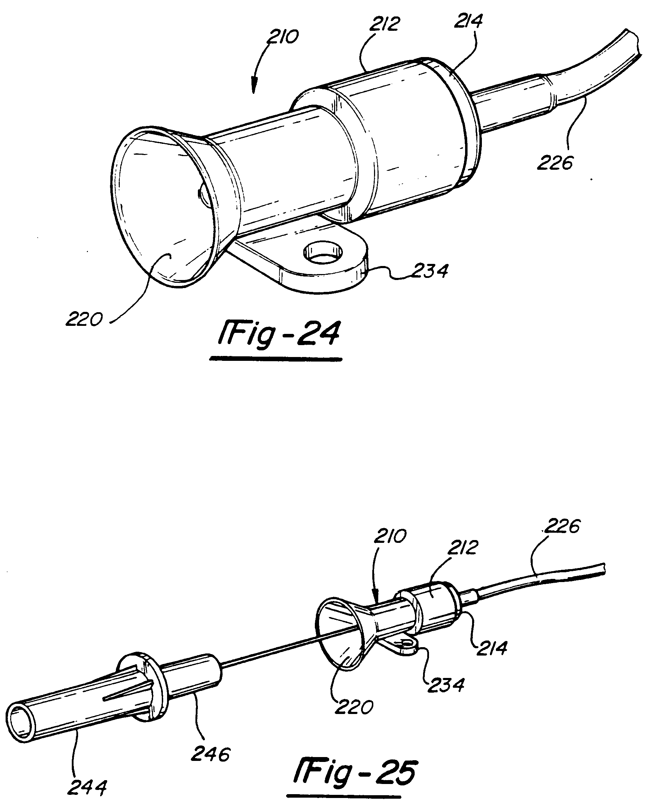

Figure 23 illustrates still another approach toward connecting outflow catheter 52 to an infusion port exit end 156. This embodiment, like that shown in Figure 20, defines an outer tubular portion 158, an inner tubular portion 160, with annular gap 162 therebetween. For this embodiment, however, the inside diameter surface of outer tubular portion 158 defines groove 164. Compression ring 166 has an exterior configuration including annular barb 168 which interlocks with groove 164 when the compression ring is slid onto exit end 156.

An infusion port shown in Figure 24 is generally designated there by reference number 210. Infusion port 210 principally comprises housing 212, outlet plug 214, and articulating valve assembly 216.

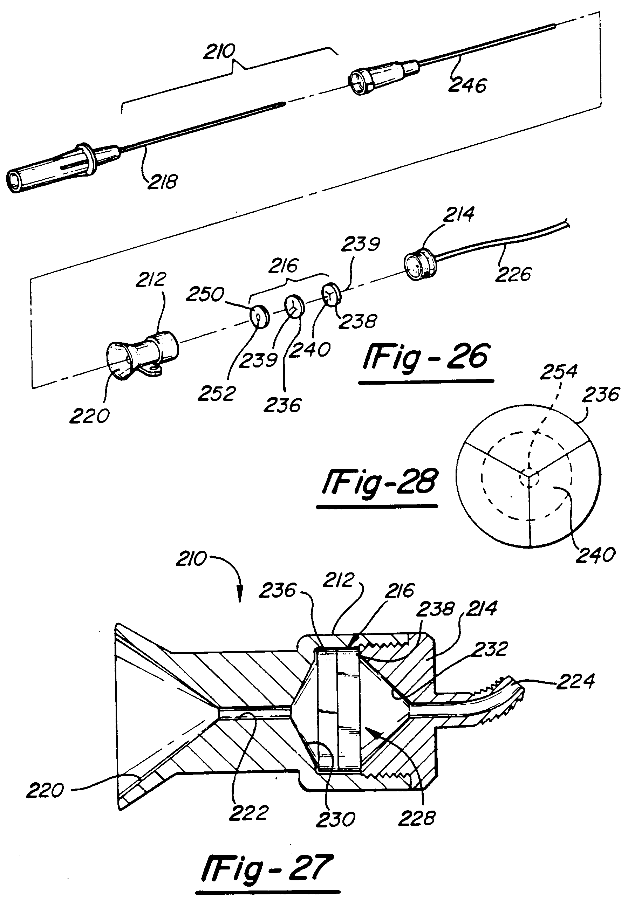

As best shown in Figures 24 and 27, housing 212 defines a funnel-shaped entrance orifice 220, the function of which is to guide an access needle 218 toward its center. Although orifice 220 is shown in the shape of a circular cone, other configurations could be used such as elliptical or flattened cones could be used to define the orifice opening. Such alternative shapes could be used to decrease the profile height of the device. Any configurations used for orifice 220 must posses a decreasing cross-sectional area for the purpose of guiding the access needle to a focus point. At the base of the orifice cavity shown in Figure 27 is a reduced diameter guide passageway 222. Guide passageway 222 is straight and has a diameter only slightly greater than a diameter of elements which are desired to be passed into port 210.

Outlet plug 214 is externally threaded which enables it to be attached to the end of housing 212 opposite entrance orifice 220. Outlet plug 214 defines an externally barbed projecting hollow post 224 which enables an internal outflow catheter 226 to be slid onto the post and attached to the infusion port as shown in Figures 24, 25 and 26. Hollow post 224 can be intentionally bent as shown in Figure 27 to prevent needle 218 from passing entirely through the device in which case it could damage outflow catheter 226. As is best shown in Figure 27, once assembled together, housing 212 and outlet plug 214 define an internal cavity which accommodates leaflet valve assembly 216. As shown in Figure 27, cavity 228 defines a pair of conical surfaces, with conical surface 230 joining with guide passageway 222 and conical surface 232 joining with exit plug post 224.

Mounting plate 234 is attached to housing 212 or formed by it integrally and provides a means of mounting infusion port 210 to support tissues within a patient using sutures, surgical staples, etc.

Leaflet valve assembly 216 shown in Figure 27 includes a pair of elastic leaflet valve discs 236 and 238. Each of the elastic discs include slits extending from their geometric center and radially outward toward the perimeter of the elastic disc to define three separate flaps or leaves 240. Elastic discs 236 and 238 are stacked against one another in a manner to disalign cuts 239 so that the leaves 240 of each disc overlies the cuts in the other to enhance the sealing characteristic of the leaflet valve assembly. As shown in Figure 27, when housing 212 and outlet plug 214 are assembled together, the outer periphery of elastic discs 236 and 238 are slightly compressed to provide a seal which prevents fluids from leaking around the outer edges of the elastic disc elements.

Figure 26 shows an optional disc ring valve element 250 (not shown in Figure 27) which is provided to further enhance the sealing characteristics of valve assembly 216. Disc element 250 has a hole 252 through its center, which has a diameter slightly smaller than the needle or catheter which port 210 is designed to accommodate. Valve element 250 is positioned to be the first element encountered by the access needle. This orientation is provided to prevent the apexes of leaves 240 from damaging valve disc 250 or interfering with its sealing capability.

Infusion port 210 is adapted to be accessed using a conventional hypodermic needle 218 with a sharp end, which can be hollow or solid depending on the intended application. Needle 218 can be used by itself or with an external catheter 246, which the needle is slid through so that the needle and catheter combination can be pierced through the skin and positioned into port 210 allowing the needle to be later withdrawn, leaving catheter 246 inside port 210 to provide fluid flow into or from the patient. The introduced catheter 246 can be threaded into outflow catheter 226 to any extent desired, preventing unintentional withdrawal of the introduced catheter.

Figure 25 shows infusion port 210 being accessed by a needle 18 and catheter 246 combination. When the physician desires to access port 210, needle 218 is used to pierce the patient's skin at an area adjacent the port entrance orifice 220 and the needle is pushed into the port. Entrance orifice 220 receives the sharp end of needle 218 and guides it toward and into guide passageway 222. The guide passageway then orients needle 218 and aims it to strike leaflet valve assembly 216 at the center of valve elements 236 and 238, which is the point of intersection of the cuts 239 defining leaves or flaps 240. Guide passageway 222, therefore, guides needle 218 to strike leaflet valve assembly 216 in an area where cutting or damage to the elastic disc elements is minimized since the discs are most easily penetrated at their central region where their flexibility is greatest. Elastic discs 236 and 238 are intentionally provided with three or more leaves or flaps since it is believed that a leaflet valve having a single slit defining only two leaves may not provide an acceptable resistance to damage by the penetrating needle. Such susceptibility to damage could occur since such a valve configuration is not believed to provide flaps with sufficient resiliency to bend away from the introduced needle, but would instead tend to be engaged and penetrated by the inserted needle 218, leading to the potential for physical damage to the disc elements.

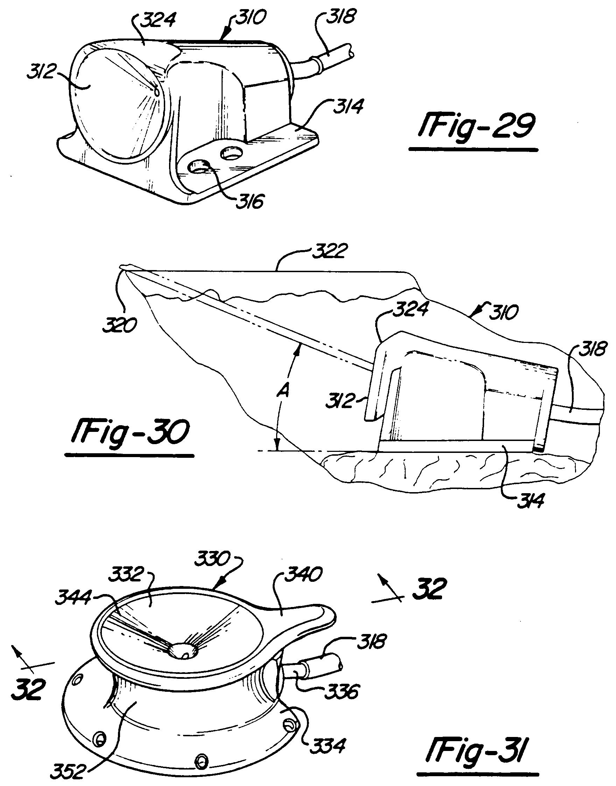

In order to provide an acceptable resistance to damage of valve assembly 216 by needle 218, it is believed that the diameter of passageway 222 which is superimposed on disc 236 in Figure 28 and designated by reference number 254, should be no larger than one-half the diameter of the slit portion of elastic discs 236 and 238 which is encompassed by a circle designated as diameter 256. Passageway diameters 254 greater than that ratio would permit a penetrating needle with its sharp point to strike the leaflet valve assembly 216 at near its outer perimeter, where leaves 240 are not as supple and are more likely to be pierced by the access needle than the center portion. Controlling the position of penetration of needle 218 also protects elastic disc 250 from damage which would occur if the needle struck outside of hole 252.

The conical surfaces 230 and 232 of valve cavity 228 are provided to accommodate the flexing of valve leaves 240 in both directions. When access needle 218 is inserted into infusion port 210, the leaves 240 are permitted to deflect toward hollow post 224. In addition, conical cavity 232 insures that the access needle 218 or other introduced filament is properly guided to pass through hollow post 224 and into internal outflow catheter 226, if desired. Upon withdrawal of access needle 218 or catheter 246 from infusion port 210, conical surface 230 enables the leaves 240 of valve assembly 216 to be freely deflected in an opposite direction.

During the step of inserting needle 218 into port 210, a positive indication of full insertion is felt by the attending physician as needle 218, which is relatively rigid, engages the bent portion of hollow post 224. This stop is provided to prevent accidental damage to outflow catheter 226. However, the introduced filament or catheter 246 which is more flexible than access needle 218 can be readily threaded past hollow post 224 to provide deep insertion.

In addition to permitting the insertion of a needle 218 and catheter 246 to port 210, this invention would allow a guide wire to be introduced into the port through access needle 218 which could be fed through the device and into and through the internal outflow catheter 246 to remove thrombosis or other clogging problems. Various other filaments type elements could also be used with port 210 such as optical fibers, electrical conductors, remote sensing systems, etc.

Numerous materials may be used to form housing 212. Since housing 212 will be subject to being struck by sharp needles which must be redirected into guide passageway 222, it is desirable to form the housing or at least the surface of orifice 220of a hard metal material such as stainless steel or titanium or a hard ceramic. Soft materials such as plastics, if used to form entrance orifice 220 could be subject to being gouged by needle 218, preventing proper guiding of the access needle. Similarly, exit outlet plug 214 is subject to being struck by a sharp needle and should also be made of a hard metal material for the reasons mentioned in connection with housing 212. Elastic discs 236, 238 and 250 can be made of numerous elastic materials such as silicone rubber.

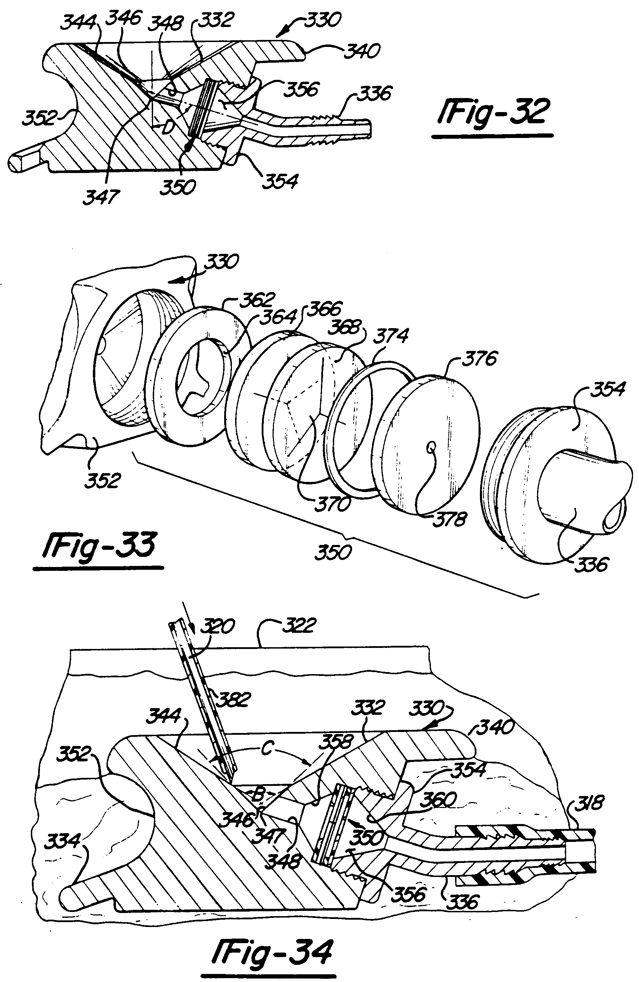

An infusion port in accordance with a seventh embodiment of this invention is shown in Figures 29 and 30 and is generally designated there by reference number 310. Port 310 is designed to be accessed using a sharp needle which passes into the port through funnel shaped entrance orifice 312. Port 310 also includes a mounting pad 314 defining a generally planer mounting surface and having apertures 316 for sutures or staples to enable the device to be secured to appropriate support tissue within the patient. Internal catheters 318 is shown attached to port 310 and is tunneled to a desired site within the patient.

The embodiment shown in Figures 29 and 30 of this invention is presented to disclose two specific improvements to devices described previously, namely a modified entrance orifice 312 and an inclination of the device with respect to mounting pad 314. As best shown in Figure 30, infusion port 310 is oriented such that the accessing needle 320 shown in phantom lines enters the device at an angle, designated as angle A from a plane parallel to mounting pad 314. The inclined orientation of port 310 facilitates insertion of needle 320 through the patients skin 322, as shown in Figure 30.

The further improvement shown in Figures 29 and 30 for infusion port 310 involves a removal of the upper surface of the housing in the area defining entrance orifice 312 shown as a scalloped region 324. Removing material in that area has the effect of slightly enlarging the target area of entrance orifice 312, and also to provide a smoother surface which is covered by the patients skin, thus making the device somewhat less conspicuous to the patient and possibly less irritating.

Although the features of infusion port 310 discussed in conjunction with Figures 29 and 30 are employed in a port of the type shown in Figure 1, these improvements could also be incorporated into ports having various constructions and internal features including other ports which are described in this application and disclosed in the related applications.

Figure 31 illustrates infusion port 330 in accordance with an eighth embodiment of this invention. Infusion port 330 is primarily intended to be implanted in the chest wall region of a patient and generally comprises a funnel shaped entrance orifice 332, mounting platform 334, outlet tube 336, and a valving system which will be described in the following description.

Mounting platform 334 features apertures 338 for enabling port 330 to be secured to underline tissue within a patient using sutures, staples, etc.

As best shown in Figure 31, infusion port housing 352 also features a radially projecting protuberance in the form of a lug or ledge 340 projecting away from entrance orifice 332, and overlying outlet tube 336. By providing such an irregular feature on the device housing 352, the orientation of the port, and in particular, outlet tube 336 and internal catheter 318 can be readily ascertained through palpation of the device by the clinician. As will be better described in the following paragraphs, for some embodiments it is necessary to cause the introduced filament to undergo a rather sharp turn upon entrance into the device, and, therefore, knowing the orientation of the port can aid in feeding in the introduced filament. Lug 340 also provides the additional benefit of shielding implanted catheter 318 from needle sticks by the accessing hypodermic needle 320, if improperly aimed.

Now with the reference to Figures 32 and 34, the configuration of entrance orifice 332 can be described in more detail. As is apparent from the figures, entrance orifice 332 is in the from of a pair of joined conical surfaces having differing cone angles. The first conical surface 344 which forms the outer perimeter of the orifice defines a relatively shallow cone having a relatively large included cone angle identified as angle B in Figure 34. Conical surface 344 joins with a smaller diameter conical surface 346 having an included angle identified as angle C in the Figure which is smaller than angle B. The shallower conical surface 344 is provided as a means of guiding inserted needle 320 toward the apex or focus area 347 of orifice 312. The relatively large angle·B of conical surface 344 is provided so that the distance through infusion port 330 between its top planer surface and the internal valve system is kept as small as reasonably possible while providing a large target area for needle 320. This total distance is significant in that presently employed catheters which are fed over needles have a relatively short length, i.e. approximately two inches. It is desirable to allow such existing needles and catheters to be used with port 330 and at the same time, insure that the introduced catheter is securely inserted into the infusion port and engaged with the internal valve. Conical surface 346 is provided with a smaller included angle as a means of securely engaging introduced needle 330 and restraining it from radial motion once it is inserted and becomes rested in focus area 347.

While the benefits of the configuration of entrance orifice 312 are achieved in accordance with the illustrated embodiment using two joined conical segments, it is fully within the scope of this invention to provide an entrance orifice defined by various other surfaces having a progressively decreasing cone angle as measured as shown in Figure 34 when moving from the outer perimeter of entrance orifice 332 to the focus area 347. For example, a paraboloid surface could also be provided for orifice 332. In addition, entrance orifice 332 could be defined by a surface which is a asymmetrical in the sense of not being a surface of revolution about an axis through the orifice. Many surfaces can be imagined providing the benefits of the invention through providing a progressively smaller cone angle or target surface as the focus area is approached.

As is shown in Figure 34 the relatively large angle of conical surface 344 serves to provide a low height between the upper surface of infusion port 330 and articulating valve 350. As mentioned previously, this is advantageous since standard introduced catheters have a relatively short length and it is desirable to make sure they are fully engaged with the articulating valve to preclude inadvertent withdrawal.

The focus area 347 of entrance orifice 332 joins with passageway 348 which leads to an articulating valve assembly 350. For reasons which will be better described later in this specification, passageway 348 is intentionally oriented with respect to the central generating axis of entrance orifice 332 at a relatively great off-axis angle, shown as angle D in Figure 32 of about 60 degrees. This off-axis orientation provides a curved passageway which is intended to prevent an introduced rigid needle 320 from undergoing the turn and directly engaging articulating valve assembly 350. This feature accordingly distinguishes infusion port 330 from the embodiments described previously which are either designed to be used with a blunt accessing instrument, or enable the inserted needle to pass directly through the articulating valve.

Housing 352 is preferably made from a hard metal material which will not be gouged or engaged by the accessing needle 320. For example, titanium or another hard metal could be used to form the entrance housing 352, or could be used merely to form the surface of entrance orifice 332.

As best shown in Figures 32 and 34, infusion port housing 352 and outlet plug 354 define catheter valve cavity 356. As shown in the Figures, cavity 356 is bounded by a pair of conical surfaces including conical surface 358 which joins with passageway 348, and conical surface 360 formed by outlet plug 354. As shown in the Figures, the included angle defined by conical surface 358 is greater than that of conical surface 360. The conical surfaces 358 and 360 are provided to enable flexing of the elements comprising articulating valve 350.

Figure 33 provides an exploded view of articulating valve assembly 350. The valve is comprised of a number of individual valve elements stacked together. The first valve element encountered when passing through valve 350 from entrance orifice 332, is a ring or donut valve 362, which is comprised of a ring of elastomeric material with a central circular aperture 364. Infusion port 330 can be used with introduced catheters of various diameters. Ring valve 362 is not provided to seal directly against the outer periphery of all sizes of introduced catheters, but rather provides a reinforcing function for the remaining catheter valve elements and also services to orient and center the introduced catheter, as will be described in more detail below. The next two valve elements are leaflet valve discs 366 and 368. Valve discs 366 and 368 each define three or more leaves 370 which form an apex at the geometric center of each valve disc. As shown in Figure 33, the leaves of each valve disc 366 and 368 are intentionally disaligned or indexed to an offset position so that the leaves are not directly overlapping. This indexing is provided to enhance the sealing capabilities of articulating valve 350. The next elements encountered in valve 350 are spacer ring 374 and finally another ring or donut valve 376 with central aperture 378. Aperture 378 has a diameter which is slightly smaller than any of the catheters which infusion port 330 is designed to be used with, thus providing a firm perimeter seal for the introduced catheters. The elements comprising articulating valve 350 are stacked together, inserted into valve cavity 356 and retained there through the threaded engagement between housing 352 and outlet plug 354.

Since hollow post 336 of outlet plug 354 is not oriented parallel to the plane defining mounting pad 314, the hollow post is bent slightly as shown in Figure 32 as a means of orientating implanted catheter 318 along the plane defining port mounting platform 334.

Figures 34 through 37 are provided to show infusion port 330 in use, and in particular, show the process of introducing an external catheter into the device. Figure 34 shows infusion port 330 implanted with a patient below the surface of skin 322. In Figure 34, a hypodermic needle 320 is shown penetrating skin 322. Needle 320 is placed through catheter 382 of conventional design such as that know as an Angiocath. Needle 320 and catheter 382 are inserted through the skin and into entrance orifice 332. Conical surface 344 initially guides the needle into conical surface 346, and finally into nesting engagement in focus area 347. As stated previously, orifice 312 is made from a material which will not be gouged by needle 320, but rather will guide it into focus area 347.

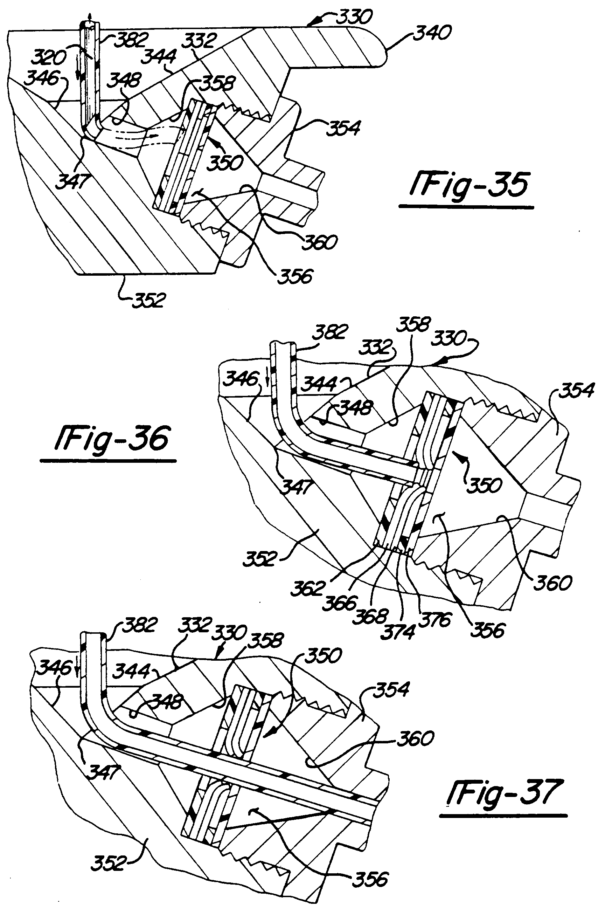

Figure 35 shows accessing needle 320 being fully inserted into focus area 347 and into passageway 348. Due to the inclination of passageway 348 from the entrance orifice, needle 320 cannot readily pass beyond the point shown in Figure 35. Once this position is reached, the clinician has positive feedback that the elements are oriented properly since it is apparent that the needle cannot be readily inserted any further into infusion port 310.

Once the point of Figure 35 is reached, the clinician can slide catheter 382 along needle 320 while holding the needle in position, thus forcing the tip of catheter 382 further into infusion port 330. Figure 35 illustrates in phantom lines that external catheters 382 undergoes a bend as it is fed into engagement with valve 350. Catheter 382 does not necessarily become oriented precisely along the longitudinal axis of passageway 348 and, therefore, does not always initially engage articulating valve assembly 350 at its center. Ring valve element 362 serves to aid in centering introduced catheter 382 to properly orient itself with respect to the remaining valve elements. As introduced catheter 382 is forced further into engagement with the catheter 350, it passes through leaflet valve discs 366 and 368. As discussed in the prior related applications, the leaves 370 can be readily opened by inserting the external catheter and their triangular shape serves to aid in centering the catheter. Finally, the introduced catheter passes through second ring valve element 376 having a relatively small aperture 378. Due to the centering functions provided by ring element 362 and the leaflet element 366 and 368, the introduced catheter becomes accurately aligned with and forced through aperture 378. Aperture 378 is sized to provide a perimeter seal around the introduced catheter 382. A fully inserted catheter is shown in Figure 37.

The design of articulating valve 350 provides a number of significant features. By providing spacing ring 374, deflection of leaflet valve leaves 370 in the direction of the insertion of catheter 382 is freely permitted. When the introduced catheter passes through the leaflet valves, leaves 370 are permitted to deflect as shown in Figures 36 and 37 without significant restriction caused by the presence of ring valve element 376. However, upon withdrawal of introduced catheter 382, reverse deflection of valve leaves 370 causes them to be reinforced by the close proximity of valve element 362, thus providing a relatively greater amount of friction during withdrawal versus insertion of catheter 382. This difference in insertion versus withdrawal friction is a desirable feature since it allows the catheter to be freely inserted into the port, yet firmly engages the inserted catheter to prevent inadvertent withdrawal of it during infusion.

The differing cone angles provided by catheter valve cavity conical surfaces 358 and 360 also provide several functions. The relatively large angle of conical surface 358 is provided to place the passageway 348 in close proximity to articulating valve 350. This enhances the "targeting" function to ensure that catheter 382 strikes the articulating valve 350 at or near its center where it can be deflected and is guided into a proper engagement with ring valve element 376. This large cone angle also serves to limit the degree of deflection of ring valve element 362, thus increasing withdrawal friction. The relatively small cone angle of conical surface 360 is provided to guide the introduced catheter smoothly into hollow post 380 and provides clearance to permit relatively unrestricted deflection of leaflet valves 366 and 368 and ring valve element 376.

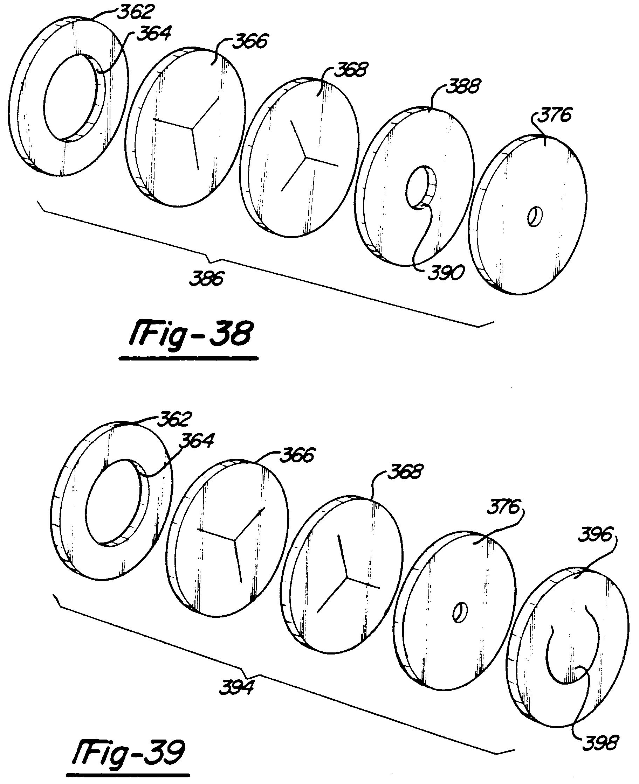

Figure 38 shows an articulating valve assembly designated by reference number 386. Articulating valve assembly 386 has a number of elements identical to articulating valve assembly 350 described previously, and the common elements are designated by common reference numbers. Articulating valve assembly 386 differs from the previous embodiment in that spacer ring 374 is replaced with another donut or ring valve element 388, having an internal circular aperture 390. The function of ring valve element 388 is to reinforce leaves 370 of valve disc 368 as a means of enhancing the sealing capabilities of articulating valve assembly 386. The diameter of aperture 390 is chosen to be larger than any introduced catheter 382 with which articulating valve assembly 386 would be used.

Figure 39 shows yet another embodiment of articulating valve assembly designated by reference number 394. This embodiment also features a number of elements common to that of articulating valve assembly 350 which are identified by like reference numbers. Articulating valve 394, however, features a flapper type valve element 396 having a central flap or leaf 398. Flapper valve 396 is provided to act as a check valve providing enhanced resistance to reverse fluid leakage since flap 398 is actuated by fluid pressure into sealing engagement with valve disc 376. Flap 398 is readily deflected upon the insertion of catheter 382 or another flexible introduced filament.

## Claims

1. An implantable patient infusion device (10) adapted to be totally implanted within the body of a patient to permit access to an internal catheter (52) either by a flexible or by a non-flexible filament (46) such as a needle (46), external catheter (32), wire or optical fibre, the device including a housing (12) having a funnel-shaped entrance orifice (14) with a decreasing cross-sectional area leading to a focus area (20), and an articulating valve (24) in a passageway (18) communicating said focus area with an exit orifice (16), said funnel-shaped entrance orifice (14) having an open area four or more times greater than the cross-sectional area of the passageway (18) and being arranged to cause the filament, when introduced into said entrance orifice, to be directed to said focus area to enter said passageway (18) and to engage said valve at a predetermined location thereon, said valve being adapted normally to remain closed to provide resistance to flow of fluids through said passageway, yet openable upon engagement with said filament to permit said filament to pass through said articulating valve and to enable said filament to communicate with an internal catheter through said exit orifice (16), said articulating valve (24) comprising at least one deflectable valve element arranged to be articulated and thereby deflected both longitudinally and laterally of the direction of movement of the filament through said articulating valve upon opening of said articulating valve by the filament.

2. An implantable patient infusion device according to claim 1, wherein said articulating valve (24) comprises a leaflet valve having a generally circular flat disk (26) of a resilient material with at least one cut (28) through said disk which crosses the centre of said disk.

3. An implantable patient infusion device according to claim 2, wherein said leaflet valve includes at least two of said disks (26) which are stacked together and are oriented so that said cuts of a first of said disks are not aligned with said cuts of a second of said valve disks.

4. An implantable patient infusion device according to claim 2 or 3, wherein said disk or disks (26) each define three or more deflectable valve leaves.

5. An implantable patient infusion device according to any one of claims 2 to 4, wherein the part of said passageway (222) joining said focus area has a diameter not greater than one half the diameter of the slit portion of said elastic disk defining said articulated leaflet valve (216).

6. An implantable patient infusion device according to any one of claims 2 to 5, wherein said housing (212) defines in said passageway an internal cavity (228) within which said leaflet valve (216) is disposed.

7. An implantable patient infusion device according to claim 5 or 6, wherein said internal cavity (228) provides clearance for the leaves of said leaflet valve (216) to deflect toward said entrance orifice (220).

8. An implantable patient infusion device according to claim 6, wherein said housing is defined by a main housing (212) defining said entrance orifice (220) and a part of said passageway (222) and an outlet plug (214) connected with said main housing (212) defining said exit orifice (224), said main housing (212) and said outlet plug (214) cooperating to define said internal cavity (228).

9. An implantable patient infusion device according to any one of claims 2 to 8, wherein said leaflet valve (216) further comprises a disk element (250,376) having a hole (252,378) for sealing against an introduced filament.

10. An implantable patient infusion device according to claim 9, wherein the disk element (376) with a hole (378) is positioned on the side of said leaflet valve (216) confronting said exit passageway (336).

11. An implantable patient infusion device according to claim 10, further comprising a spacer ring (374) placed between said leaflet valve (366,368) and said disk element (376) with a hole (378).

12. An implantable patient infusion device according to claim 9, comprising first (374) and second (376) valve elements positioned between said leaflet valve disks (366,368) and said exit orifice (336) wherein said first valve element (374) is a ring which supports said leaflet valve leaves and said second valve element (376) defines a perimeter seal around a filament, when introduced.

13. A implantable patient infusion device according to claim 1, wherein the articulating valve comprises a flapper valve element (396).

14. An implantable patient infusion device according to claim 1, wherein said articulating valve comprises a cup valve (56) having a nipple (68) with an exit side and a valve cup (62) for sealing engagement with said nipple, and means (64) for resiliently urging said cup to zeal against said nipple, said cup valve being oriented such that said filament will unseat said cup from said nipple when introduced into said infusion device.

15. An implantable patient infusion device according to claim 1, wherein said articulating valve comprises a ball-and-seat type valve (68) wherein said ball (72) sealingly engages with a seat (70) formed by said infusion device and said ball is urged to unseat from said seat when said filament is introduced into said infusion device.

16. An implantable patient infusion device according to any one of claims 1 to 5, wherein said infusion device (94) comprises at least two of said articulating valves (24) positioned within said housing (12) and separated to define an antimicrobial fluid reservoir (98).

17. An implantable patient infusion device according to any preceding claim, wherein said infusion device further comprises an elastic septum (31) covering said entrance orifice and having a preformed slit (34) therethrough.

18. An implantable patient infusion device according to claim 1, wherein said articulating valve (24) means imposes less friction upon said filament being inserted through said valve than imposed upon said filament upon withdrawal of said filament.

19. An implantable patient infusion device according to claim 18, wherein said articulating valve (24) includes at least one leaflet valve element having leaves which deflect when said filament is placed through said valve, and means for allowing said leaves to deflect more readily in the direction of insertion of said filament as compared with the direction of removal of said filament from said device.

20. An implantable patient infusion device according to any preceding claim, wherein said housing entrance (12) orifice is formed from a hard material (312) whereby a needle contacting said surface is guided into said focus area.

21. An implantable patient infusion device according to claim 20, wherein said housing defines stop means (224,346) to limit the amount of penetration of said needle in said housing after passing through said valve while allowing more flexible introduced filaments (382) to be threaded entirely through said device.

22. An implantable patient infusion device according to claim 21, wherein said stop means comprises a curved passageway (224) which defines the limit of insertion.

23. An implantable patient infusion device according to claim 22, wherein said curved passageway is adapted to allow a filament to be pushed therethrough and into an implanted internal catheter connected to said exit orifice.

24. An implantable patient infusion device according to claim 20, further comprising stop means (346) within said passageway between said focus area and said articulating valve for restricting the passage of said needle (382) while permitting said introduced filament to pass through said passageway and engage said articulating valve.

25. An implantable patient infusion device according to claim 24, wherein said stop means comprises a bend (346) in said passageway.

26. An implantable patient infusion device according to claim 20, further comprising an external catheter (382) having said access needle (320) inserted into its hollow core and said infusion device allowing the combination of said needle and said external catheter to be inserted into said device and thereafter allowing said needle to be removed leaving said external catheter inside said device.

27. An implantable patient infusion device according to claim 1, wherein said entrance orifice defines a surface (332) having a first included cone angle (c) around its outside perimeter and defining a second included cone angle (b) adjacent said focus area (346) which is smaller than said first included cone angle (c).

28. An implantable patient infusion device according to claim 27, wherein said first and second included cone angles are formed by joined conical surfaces (344,346).

29. An implantable patient infusion device according to claim 1, wherein said housing entrance orifice (14) has a central axis generally coaxial with the exit orifice (16).

30. An implantable patient infusion device according to claim 1, wherein said entrance orifice defines a generally circular perimeter (344) lying on a plane positioned such that a line normal to said plane forms a right angle to the central axis of said exit orifice (336) with said housing passageway (346,348) being shaped to guide said filament inserted into said entrance orifice to bend and become inserted through said articulating valve (350).

31. An implantable patient infusion device according to claim 30, wherein said housing entrance orifice has a central axis adapted to be located generally perpendicular to the patient's skin and further comprises a projection (340) which can be detected by external palpation after said device has been implanted to indicate the orientation of said device.

32. An implantable patient infusion device according to claim 1, wherein said entrance orifice (84) defines a plane which is oriented such that a line normal to said plane forms an obtuse angle with respect to a central longitudinal axis through said exit orifice (86).

## Patentansprüche

1. Eine einem Patienten implantierbare Infusionsvorrichtung (10), die so beschaffen ist, daß sie vollständig innerhalb des Körpers eines Patienten implantiert werden kann, um Zugang zu einem internen Katheter (52) entweder durch einen flexiblen oder durch einen nicht-flexiblen Elementarfaden (46), wie z.B. eine Nadel (46), einen externen Katheter (32), einen Draht oder eine Lichtleitfaser zu ermöglichen, wobei ein Gehäuse (12), das eine trichterförmige Eingangsöffnung (14) mit einer sich verringerenden Querschnittsfläche hat, die zu einer Brennpunktfläche (20) führt, zur Vorrichtung gehört, sowie ein gegliedertes Ventil (24) in einem Durchgang (18), der die genannte Brennpunktfläche mit einer Ausgangsöffnung (16) verbindet, wobei die genannte trichterförmige Eingangsöffnung (14) eine Öffnungsfläche hat, die vier oder noch mehr mal so groß wie die Querschnittsfläche der Durchgangsöffnung (18) ist und die so angeordnet ist, daß sie es bewirkt, daß der Elementarfaden, nachdem er in die genannte Eingangsöffnung eingeführt worden ist, zur genannten Brennpunktfläche gelenkt wird, um in den Durchgang (18) eingeführt zu werden und auf das genannte Ventil an einer vorher bestimmten Stelle aufzutreffen, wobei das genannte Ventil normalerweise so beschaffen ist, daß es geschlossen bleibt, um dem Durchfluß von Flüssigkeit durch die genannte Durchgangsöffnung zu widerstehen, aber daß es nach dem Eingriff des genannten Elementarfadens geöffnet werden kann, um es dem genannten Elementarfaden zu ermöglichen, durch das genannte gegliederte Ventil zu passieren und es dem genannten Elementarfaden zu ermöglichen, mit einem internen Katheter durch die genannte Ausgangsöffnung (16) in Vergindung zu treten, wobei das genannte gegliederte Ventil (24) aus wenigstens einem biegbaren Ventilteil besteht, das so beschaffen ist, daß es gelenkig angebracht werden kann und dadurch sowohl in der Richtung der Bewegung als auch seitlich zur Bewegungsrichtung des Elementarfadens durch das genannte gegliederte Ventil abgelenkt werden kann, wenn das genannte gegliederte Ventil durch den Elementarfaden geöffnet wird.

2. Eine einem Patienten implantierbare Infusionsvorrichtung nach Anspruch 1, in der das genannte gegliederte Ventil (24) aus einem Klappenventil besteht, das aus einer im allgemeinen kreisförmigen flachen Scheibe (26) besteht mit wenigstens einem Schnitt (28) durch die genannte runde Scheibe, welcher durch den Mittelpunkt der genannten runden Scheibe verläuft.

3. Eine einem Patienten implantierbare Infusionsvorrichtung nach Anspruch 2, in der das genannte Klappenventil wenigsten zwei der genannten runden Scheiben (26) enthält, die aufeinander geschichtet sind und so ausgerichtet sind, daß die genannten Schnitte einer ersten dieser runden Scheiben nicht mit den besagen Schnitten einer zweiten der genannten runden Ventilscheiben ausgerichtet sind.

4. Eine einem Patienten implantierbare Infusionsvorrichtung nach den Ansprüchen 2 oder 3, in der die genannte runde Scheibe oder die runden Scheiben (26) jede drei oder mehr biegbare Ventilklappen bilden.

5. Eine einem Patienten implantierbare Infusionsvorrichtung nach jedem der Patentansprüche 2 bis 4, in der der Teil des genannten Durchgangs (222), der mit der genannten Brennpunktfläche in Verbindung steht, einen Durchmesser hat, der nicht größer als die Hälfte des Durchmessers des geschlitzten Teils der genannten elastischen runden Scheibe ist, die das gegliederte Klappenventil (216) formt.

6. Eine einem Patienten implantierbare Infusionsvorrichtung nach einem der Patentansprüche 2 bis 5, in der das genannte Gehäuse (212) in dem genannten Durchgang einen inneren Hohlraum (228) bildet, in dem das genannte Klappenventil (216) angeordnet ist.

7. Eine einem Patienten implantierbare Infusionsvorrichtung nach den Ansprüchen 5 oder 6, in der der genannte innere Hohlraum (228) für einen Abstand für die Ventilklappen des genannten Klappenventils (216) sorgt, um sie in der Richtung zur genannten Eingangsöffnung (220) abzulenken.

8. Eine einem Patienten implantierbare Infusionsvorrichtung nach Anspruch 6, in der das genannte Gehäuse durch ein Hauptgehäuse (212) gebildet wird, das die genannte Eingangsöffnung (220), einen Teil des genannten Durchgangs (222) und einen Ausgangsstöpsel (214), der mit dem genannten Hauptgehäuse (212) verbunden ist, wodurch die genannte Ausgangsöffnung (224) geformt wird und wo das genannte Hauptgehäuse (212), und der genannte Ausgangsstöpsel (214) so miteinander zusammenwirken, daß sie den inneren Hohlraum (228) formen.

9. Eine einem Patienten implantierbare Infusionsvorrichtung nach jedem der Patentansprüche 2 bis 8, in der das genannte Klappenventil (216) auch noch ein weiteres Rundscheibenelement (250, 376) enthält, das mit einem Loch (252, 378) versehen ist, um es gegen einen eingeführten Elementarfaden abzudichten.

10. Eine einem Patienten implantierbare Infusionsvorrichtung nach Anspruch 9, in der das mit einen Loch (378) versehene Rundscheibenelement (376) an der Seite des genannten Klappenventils (216) positioniert ist und dem genannten Ausgangsdurchgang (336) gegenüber liegt.

11. Eine einem Patienten implantierbare Infusionsvorrichtung nach Anspruch 10, die auch noch einen ringförmigen Abstandhalter (374) enthält, der zwischen dem genannten Klappenventil (366, 368) und dem genannten mit einem Loch (378) versehenen Rundscheibenelement (376) gelegen ist.

12. Eine einem Patienten implantierbare Infusionsvorrichtung nach Anspruch 9, die erste (374) und zweite (376) Ventilelemente enthält, die zwischen den genannten Klappenventilscheiben (366, 368) und der genannten Ausgangsöffnung (336) positioniert sind, in der das erste Ventilelement (374) ein Ring ist, der die genannten Klappenventilklappen stützt, und in der das genannte zweite Ventilelement (376) eine Perimeterdichtung um den Elementarfaden herum bildet, wenn er eingeführt wird.

13. Eine einem Patienten implantierbare Infusionsvorrichtung nach Anspruch 1, in der das gegliederte Ventil ein Klappenventilelement (396) enthält.

14. Eine einem Patienten implantierbare Infusionsvorrichtung nach Anspruch 1, in der das genannte gegliederte Ventil aus einem Tassenventil (56) besteht, das einen Nippel (68) enthält mit einer Ausgangsseite und einer Ventiltasse (62), um es gegen den genannten Nippel abzudichten, und eine Vorrichtung (64), um die genannte Tasse elastisch gegen den genannten Nippel zum Abdichten zu drücken, wobei das genannte Tassenventil so ausgerichtet ist, daß die genannte Elementarfaser die genannte Tasse von dem genannten Nippel abheben wird, wenn sie in die Infusionsvorrichtung eingeführt wird.

15. Eine einem Patienten implantierbare Infusionsvorrichtung nach Anspruch 1, in der das genannte gegliederte Ventil aus einem aus Ball und Sitzfläche bestehendem Ventil (68) besteht, in dem der genannte Ball (72) mit der Sitzfläche (70) eine Dichtung bildet, die von der genannten Infusionsvorrichtung gebildet wird, und der genannte Ball wird dazu bewegt, sich von der genannten Sitzfläche abzuheben, wenn die genannte Elementarfaser in die genannte Infusionsvorrichtung eingeführt wird.

16. Eine einem Patienten implantierbare Infusionsvorrichtung nach einem der Ansprüche 1 bis 5, wobei die genannte Infusionsvorrichtung (94) wenigstens zwei der genannten gegliederten Ventile (24) enthält, die innerhalb des genannten Gehäuses (12) positioniert sind und von einander getrennt sind, um ein Flüssigkeitsreservoir (98) für eine antimikrobielle Flüssigkeit zu bilden.

17. Eine einem Patienten implantierbare Infusionsvorrichtung nach jedem der vorhergehenden Ansprüche, wobei die genannte Infusionsvorrichtung auch noch ein elastisches Septum (31) enthält, das die Eingangsöffnung bedeckt und durch das ein vorgeformter Schlitz (34) sich erstreckt.

18. Eine einem Patienten implantierbare Infusionsvorrichtung nach Anspruch 1, in der die genannte gegliederte Ventilvorrichtung (24) eine geringere Reibung auf die genannte Elementarfaser, die durch das genannte Ventil eingeführt wird, ausübt als die Reibung, die auf die genannte Elementarfaser während des Herausziehens der genannten Elementarfaser ausgeübt wird.

19. Eine einem Patienten implantierbare Infusionsvorrichtung nach Anspruch 18, in der das genannte gegliederte Ventil (24) wenigstens ein Klappenventilelement enthält, das mit Ventilklappen versehen ist, die abgelenkt werden, wenn die genannte Elementarfaser durch das genannte Ventil geführt wird, und eine Vorrichtung, die es ermöglicht, daß die genannten Ventilklappen leichter in der Richtung der Einführung der genannten Elementarfaser abgelenkt werden können als in der Richtung, in der die genannte Elementarfaser von der genannten Vorrichtung herausgezogen wird.

20. Eine einem Patienten implantierbare Infusionsvorrichtung nach jedem der vorhergehenden Ansprüche, in der die genannte Gehäuseeingangsöffnung (12) von einem harten Material (312) geformt wird, wodurch ein Stift, der mit der genannten Fläche in Berührung kommt, zur genannten Brennpunktfläche gelenkt wird.

21. Eine einem Patienten implantierbare Infusionsvorrichtung nach Anspruch 20, in der das genannte Gehäuse eine Stoppvorrichtung (224, 346) formt, um die Tiefe des Eindringens des genannten Stifts in das genannte Gehäuse zu begrenzen, nachdem der Stift das genannte Ventil passiert hat, während es den flexibler eingeführten Elementarfasern (382) ermöglicht, vollständig durch die genannte Vorrichtung gefädelt zu werden.

22. Eine einem Patienten implantierbare Infusionsvorrichtung nach Anspruch 21, in der die genannte Stoppvorrichtung einen gekrümmten Durchgang (224) enthält, der die Grenzen der Einführung definiert.

23. Eine einem Patienten implantierbare Infusionsvorrichtung nach Anspruch 22, in der der genannte gekrümmte Durchgang so ausgebildet ist, daß er es einer Elementarfaser ermöglicht, durch ihn hindurch und in einen implantierten Katheter hinein geschoben zu werden, der mit der genannten Ausgangsöffnung verbunden ist.

24. Eine einem Patienten implantierbare Infusionsvorrichtung nach dem Anspruch 20, der auch eine Stoppvorrichtung (346) innerhalb dem genannten Durchgangs zwischen der Brennpunktfläche und dem genannten gegliederten Ventil enthält, um den Durchgang für den genannten Stift (382) zu beschränken, während sie es der eingeführten Elementarfaser erlaubt, durch den Durchgang zu passieren und mit dem genannten gegliederten Ventil in Berührung zu kommen.

25. Eine einem Patienten implantierbare Infusionsvorrichtung nach Anspruch 24, in der die genannte Stoppvorrichtung eine Krümmung (346) in dem genannten Durchgang enthält.

26. Eine einem Patienten implantierbare Infusionsvorrichtung nach Anspruch 20, die auch noch einen externen Katheter (382) enthält, bei dem der genannte Zugangsstift (320) in seinen hohlen Kern eingeführt wird, und bei dem die genannte Infusionsvorrichtung es der Kombination, die aus dem genannten Stift und dem genannten externen Katheter besteht, erlaubt, in die genannte Vorrichtung eingeführt zu werden, und es danach dem genannten Stift ermöglicht, entfernt zu werden, wobei der genannte externe Katheter innerhalb der Vorrichtung verbleibt.

27. Eine einem Patienten implantierbare Infusionsvorrichtung nach Anspruch 1, in der die genannte Eingangsöffnung eine Oberfläche (332) bildet, die einen ersten eingeschlossenen Kegelwinkel (c) um ihren äußeren Umfang herum hat, und wobei ein zweiter eingeschlossener Kegelwinkel (b) neben der genannten Brennpunktfläche (346) gebildet wird, der kleiner als der erste eingeschlossene Kegelwinkel (c) ist.

28. Eine einem Patienten implantierbare Infusionsvorrichtung nach Anspruch 27, in der die ersten und die zweiten eingeschlossenen Kegelwinkel durch miteinander verbundene Kegeloberflächen (344, 346) gebildet werden.

29. Eine einem Patienten implantierbare Infusionsvorrichtung nach Anspruch 1, in der die genannte Eingangsöffnung (14) zum Gehäuse eine Mittleachse hat, die im allgemeinen koaxial mit der Ausgangsöffnung (16) ist.

30. Eine einem Patienten implantierbare Infusionsvorrichtung nach Anspruch 1, in der die genannte Eingangsöffnung einen allgemein kreisförmigen Umfang (344) formt, der in einer Ebene liegt, die so positioniert ist, daß eine Linie, die senkrecht zu der genannten Ebene verläuft, einen rechten Winkel zur Mittelachse der genannten Ausgangsöffnung (336) bildet, wobei der genannte Gehäusedurchgang (346, 348) so geformt ist, daß er die genannte Elementarfaser, die in die genannte Eingangsöffnung eingeführt ist, so steuert, daß sie gebogen wird und durch das genannte gegliederte Ventil (350) eingeführt wird.

31. Eine einem Patienten implantierbare Infusionsvorrichtung nach Anspruch 30, in der die genannte Eingangsöffnung zum Gehäuse eine Mittelachse hat, die so ausgerichtet ist, daß sie allgemein senkrecht zur Haut des Patienten gelegen ist und auch noch einen Vorsprung (340) formt, der durch externes Abklopfen entdeckt werden kann, nachdem die genannte Vorrichtung implantiert worden ist, um die Orientierung der genannten Vorrichtung anzuzeigen.

32. Eine einem Patienten implantierbare Infusionsvorrichtung nach Anspruch 1, in der die genannte Eingangsöffnung (84) eine Ebene bildet, die so orientiert ist, daß eine Linie, die senkrecht zu der genannten Ebene liegt, einen stumpfen Winkel formt mit Hinsicht auf eine zentrale Längsachse durch die genannte Ausgangsöffnung (86). können als in der Richtung, in der die genannte Elementarfase

## Revendications

1. Un dispositif d'infusion interne (10) prévu pour être complètement implanté dans le corps du patient afin de donner accès à une sonde interne (52), soit à l'aide d'un filament flexible ou rigide tel qu'une aiguille (46), une sonde externe (32), un fil, ou bien une fibre optique, ledit dispositif comprenant un logement (12) à orifice d'entrée conique (14) qui s'effile progressivement pour aboutir à une zone focale (20), ainsi qu'un clapet d'articulation (24) situé dans un passage (18) qui relie ladite zone focale à un orifice de sortie (16), ledit orifice d'entrée conique (14) ayant un diamètre d'au moins quatre fois celui du passage (18) et disposé de telle manière à diriger le filament introduit dans ledit orifice vers la zone focale et dans le passage (18), engageant ledit clapet à un point déterminé, ledit clapet étant normalement fermé afin de créer une résistance au débit des fluides traversant le passage, sauf lorsqu'il est ouvert par l'engagement dudit filament, permettant audit filament de traverser ledit clapet d'articulation pour permettre audit filament de communiquer avec une sonde interne via ladite orifice de sortie (16), ledit clapet d'articulation (24) comprenant au moins un élément dérivable articulé lui permettant d'être dérivée à la fois longitudinalement et latéralement dans la direction du filament à travers ledit clapet d'articulation lors de l'ouverture dudit clapet d'articulation par le filament.

2. Un dispositif d'infusion interne selon la première revendication, caractérisé par le fait que ledit clapet d'articulation (24) est équipé d'une valvule dont le disque est généralement rond et plat (26) et fait d'un matériaux résiliant, pourvue d'au moins une entaille (28) qui traverse le centre dudit disque.

3. Un dispositif d'infusion interne selon la revendication numéro 2, où ladite valvule est équipée d'au moins deux desdits disques (26), empilés et disposés de telle manière à ce que lesdites entailles du disque précédent sont décalées par rapport à celles du disque suivant.

4. Un dispositif d'infusion interne selon les revendications 2 ou 3, où un ou plusieurs desdits disques (26) représentent au moins trois valvules dérivables.

5. Un dispositif d'infusion interne selon l'une quelconque des revendications 2 à 4 où la partie dudit passage (222) reliée à ladite zone focale a un diamètre maximal égal à la moitié du diamètre de la partie entaillée dudit disque élastique représentant ladite valvule (216).

6. Un dispositif d'infusion interne selon l'une quelconque des revendications 2 à 5 où ladite gaine (212) représente, à l'intérieur dudit passage, un vide (228) dans lequel se trouve ladite valvule (216).

7. Un dispositif d'infusion interne selon l'une des revendications 5 ou 6 où le vide (228) permet aux feuilles dudit clapet à valvules (216) d'être déviées vers ledit orifice d'entrée (220).

8. Un dispositif d'infusion interne selon la revendication 6 où ladite gaine est représentée par une gaine principale (212) qui représente ledit orifice (220), ainsi qu'une partie dudit passage (222) et d'un bouchon de sortie (214) lié à la gaine principale (212) représentant ledit orifice de sortie (224), avec ladite gaine principale (212) et ledit bouchon de sortie (214) s'unissant pour représenter ledit vide interne (228).

9. Un dispositif d'infusion interne selon l'une des revendications 2 à 8 où ledit clapet à valvule (216) comprend également un disque (250, 376) pourvu d'un trou (252, 378) assurant l'étanchéité d'un filament introduit.

10. Un dispositif d'infusion interne selon la revendication numéro 9 où le disque (376) avec trou (378) est disposé sur le côté dudit clapet à valvule (216), face au passage de sortie (336).

11. Un dispositif d'infusion interne selon la revendication numéro 10 comprenant aussi une bague d'écartement (374) positionnée entre ledit clapet à valvule (366, 368) et le disque (376) à trou (378).

12. Un dispositif d'infusion interne selon la revendication numéro 9 comprenant un clapet principal (374) et un clapet secondaire (376) positionnés entre lesdits disques de clapet à valvule (366, 368) et ledit orifice de sortie (336) où le premier desdits clapets (374) est composé d'une bague qui soutient lesdites feuilles de clapet à valvule et le second desdits clapets (376) assure l'étanchéité périphérique du filament lorsque celui-ci est introduit.

13. Un dispositif d'infusion interne selon la première revendication, où le clapet articulé comprend un clapet à battant (396).

14. Un dispositif d'infusion interne selon la première revendication, où ledit clapet articulé comprend un clapet à ventouse (56) à tétine (68) avec sortie et une ventouse de valvule (62) permettant l'étanchéité de l'engagement avec ladite tétine, ainsi qu'un moyen (64) d'inciter l'étanchéité flottante de ladite ventouse avec ladite tétine, ledit clapet à ventouse étant orienté de manière à ce que ledit filament puisse déloger ladite ventouse de clapet lorsqu'il est introduit audit dispositif d'infusion.

15. Un dispositif d'infusion interne selon la première revendication, où ledit clapet articulé comporte un clapet à bille (68) dans lequel ladite bille (72) s'engage de manière étanche dans un logement (70) formé par ledit dispositif d'infusion et où ladite bille se déloge dudit logement lorsque ledit filament est introduit dans ledit dispositif d'infusion.

16. Un dispositif d'infusion interne selon une quelconque des revendications 1 à 5, où ledit dispositif d'infusion (94) comporte au moins deux desdits clapets (24) positionnés à l'intérieur dudit logement (12) et séparés de manière à créer un réservoir de fluide antimicrobien (98).

17. Un dispositif d'infusion interne selon une quelconque des revendications précédentes, où ledit dispositif d'infusion comporte également une cloison élastique (31) avec entaille préformée (34) recouvrant ledit orifice d'entrée.

18. Un dispositif d'infusion interne selon la première revendication, où ledit clapet articulé (24) offre moins de résistance lorsque ledit filament est introduit dans ledit clapet que lorsque ledit filament en est retiré.

19. Un dispositif d'infusion interne selon la revendication 18, où ledit clapet articulé (24) comprend au moins une valvule équipée de feuilles qui se détournent lorsque ledit filament est introduit dans ledit clapet, ainsi que d'un moyen de permettre aux dites feuilles de se détourner plus facilement dans le sens d'insertion dudit filament que dans le sens de retrait dudit filament.

20. Un dispositif d'infusion interne selon une quelconque des revendications précédentes, où l'orifice d'entrée dudit logement (12) est composé d'une matière dure (312) permettant de guider une aiguille venant en contact avec ladite surface vers ladite zone focale.

21. Un dispositif d'infusion interne selon la revendication 20, où ledit logement crée un moyen de butée (224, 346) permettant de limiter la progression de ladite aiguille après son passage à travers dudit clapet, tout en permettant aux filaments souples (382) d'être complètement enfilés à travers ledit dispositif.

22. Un dispositif d'infusion interne selon la revendication 21, où ledit moyen de butée comprend un passage recourbé (224) qui délimite la profondeur d'insertion.

23. Un dispositif d'infusion interne selon la revendication 22, où ledit passage recourbé est conçu de telle manière que l'on puisse pousser un filament à travers ledit passage et dans une sonde interne raccordée audit orifice de sortie.

24. Un dispositif d'infusion interne selon la revendication 20, comprenant en outre un moyen de butée (346) disposé à l'intérieur dudit passage, entre ladite zone focale et ledit clapet articulé, afin de limiter le trajet de ladite aiguille (382), tout en permettant audit filament introduit de franchir ledit passage et de s'engager dans le clapet articulé.

25. Un dispositif d'infusion interne selon la revendication 24, où ledit moyen de butée est composé d'une courbe (346) dans ledit passage.

26. Un dispositif d'infusion interne selon la revendication 20, comprenant en outre une sonde externe (382) dont ladite aiguille d'accès (320) est introduite dans son âme creuse, ledit dispositif d'infusion permettant alors l'ensemble composé de ladite aiguille et de ladite sonde externe d'être introduit dans ledit dispositif, pour ensuit permettre à ladite aiguille d'être retirée, laissant seule ladite sonde externe à l'intérieur dudit dispositif.

27. Un dispositif d'infusion interne selon la première revendication, où ledit orifice d'entrée représente une surface (332) incorporant un premier angle de cône périmétrique (c) et un second angle de cône (b) qui est plus petit que l'angle de cône (c), situé au droit de ladite zone focale (346).

28. Un dispositif d'infusion interne selon la revendication 27, où lesdits premier et second angles de cône incorporés sont formés par des surfaces coniques reliées (344, 346).

29. Un dispositif d'infusion interne selon la première revendication, où ledit orifice d'entrée du logement (14) a un axe qui est généralement coaxial par rapport à celui de l'orifice de sortie (16).

30. Un dispositif d'infusion interne selon la première revendication, où ledit orifice d'entrée décrit un périmètre généralement circulaire (344) reposant sur un plan disposé perpendiculairement à l'axe central dudit orifice de sortie (336), ledit passage du logement (346, 348) ayant une forme qui permet de guider le filament introduit dans ledit orifice d'entrée à travers la courbe, pour ensuite passer à travers le clapet articulé (350).

31. Un dispositif d'infusion interne selon la revendication 30, où l'axe central de l'orifice d'entrée dudit logement est généralement perpendiculaire à la peau du patient et comprend en outre une projection (340) pouvant être détectée par palpation externe une fois que ledit dispositif a été implanté, permettant ainsi de déterminer l'orientation dudit dispositif.

32. Un dispositif d'infusion interne selon la première revendication, où l'orifice d'entrée (84) constitue une surface orienté de telle manière qu'elle forme un angle obtus par rapport à l'axe central longitudinal dudit orifice (86).
